# EUROPEAN PATENT APPLICATION

(11) **EP 4 464 364 A1**
(43) Date of publication of application: **20.11.2024**
(21) Application number: 24176739.1
(22) Date of filing: 17.05.2024
(51) Int. Cl.: A61M 60/139, A61M 60/295, A61M 60/497, A61M 60/531, A61M 60/843

(54) **SYSTEM AND METHOD FOR PREVENTING GAS LEAKAGE FROM AN INTRA-AORTIC BALLOON**

(30) Priority: 19.05.2023 US 202318199671
(71) Applicant: NuPulseCV, Inc., Raleigh, NC 27607 (US)
(72) Inventor: Ivers, Douglas Edward, Cary (US)
(74) Representative: Forresters IP LLP

(57) **Abstract**

A system for limiting inflation of an expandable member (e.g., a balloon) in an intravascular circulatory support system of a patient may comprise a drive unit and an air mover limiter. The drive unit may include an air mover and a motor. The air mover may have a first end and a second end. The first end may be fixed and have a pneumatic output in fluid connection with the expandable member. The second end may be movable and pneumatically closed. The air mover limiter may be configured to restrict displacement of the second end to under-inflate the expandable member based on the blood pressure of the patient. Displacement of the second end moves a volume of air into or out of the expandable member. The volume of air corresponds to the air mover limiter configuration.

## Description

### TECHNICAL FIELD

The present technology is directed to systems and devices for treating heart failure, and in particular to a drive unit configured to prevent leaks in intravascular circulatory support systems.

### BACKGROUND

The prevalence of heart failure is increasing worldwide and is an expensive burden on health care providers. Despite advances in medical care, prognosis for patients with heart failure remains poor, especially in patients having advanced stage heart failure. This is due in part to limited therapy options for such patients. Counterpulsation using an intravascular circular support system is a treatment option for heart failure. Such a system may include a thin, flexible tube called a catheter with a long, thin balloon attached to the catheter tip. The balloon is positioned in the aorta of a patient. The other end of the catheter may attach to a computer console or "drive unit" with a mechanism for inflating and deflating the balloon at the proper time during the heartbeat. The balloon acts as a balloon pump (i.e., an intra-aortic balloon pump (IABP)). The drive unit may inflate the balloon when the heart relaxes to push blood towards the end-organs and the coronary arteries to perfuse the heart. And before the left ventricle contracts, the drive unit may cause the balloon to deflate, reducing the pressure that the heart has to pump against. This enables the heart to pump more blood into the body while using less energy. The drive unit may continue to inflate and deflate the balloon in sync with the heartbeat. One system that employs counterpulsation is described in U.S. Pat. No. 3,266,487 entitled "Heart Pump Augmentation System and Apparatus" filed on June 4, 1963 to Watkins et al. This patent generally discloses an intra-aortic balloon pump (IABP) using reservoirs of high and low pressure air to inflate and deflate the balloon.

Drive units may employ helium, air, or other gasses or fluids to inflate and deflate the balloon. There are technical costs, patient usability and portability issues, and safety risks associated with using IABP. For example, in the event of a balloon leak or rupture, helium is not readily absorbed in blood and poses a significant thrombogenic and stroke risk to the patient. To a lesser degree, systems employing air or other fluids also present similar risks in the event of balloon leaks or ruptures.

Past IABP counterpulsation systems generally describe full inflation of the balloon and cannot detect partial balloon inflation:
- U.S. Pat. No. 3,720,199 entitled "Safety Connector for Balloon Pump" filed on May 14, 1971 to Rishton et al. ("the '199 patent") describes venting gas if the IABP should become over-inflated. If the measured IABP pressure exceeds a limit, a valve that is external to the patient opens and vents gas to the atmosphere. The '199 patent also describes that IABP under-inflation may form thrombosis.
- U.S. Pat. No. 5,817,001 entitled "Method and Apparatus for Driving an Intra-Aortic Balloon Pump" filed on May 27, 1997 to Leschinsky et al. describes a two-phased balloon inflation step. First, the external driver applies increased gas pressure into the balloon for a brief time in order to expedite initial inflation, but then describes a second, "dwell" phase that reduces positive gas pressure to a "normal" inflation pressure to prevent over-pressurizing the balloon.
- U.S. Patent 6,735,532 entitled "Cardiovascular Support Control System" filed on November 18, 2002 to Freed et al. generally describes a "partial cycle" that restricts balloon inflation for a few cardiac cycles to measure patient arterial pressure. Pressure sensors in the external driver may then measure timing of the dicrotic notch during the patient's cardiac cycle. The external driver synchronizes balloon inflation with the patient's dicrotic notch timing. After the "partial cycle" measurement is completed, the system returns to alternately fully inflating and fully deflating the balloon.
- U.S. Pat. No. 5,513,956 generally describes accommodating different balloon sizes by controlling the degree of balloon inflation. During a counterpulsation inflation step, the balloon is fully inflated.

Past systems have also described detecting IABP leaks, but only after gas has escaped from the balloon, significantly increasing the risk of patient harm:
- U.S. Pat. No. 5,513,956 entitled "Circulatory Assisted Device with Motor Driven Gas Pump" filed on January 14, 1994 to Lewis et al. generally describes a method for leak checking in a gas management subsystem, but only when the counterpulsation system is outside of the patient.
- U.S. Pat. No. 6,536,260 entitled "Balloon Catheter Leak Detection Method and Apparatus" filed on July 11, 2001 to Williams and U.S. Pat. No. 6,735,532 entitled "Cardiovascular Support Control System" filed on November 18, 2002 to Freed et al. generally describe leak detection by monitoring the pressure across multiple counterpulsation cycles at the same point in the cycle. If the pressure drops past a preset threshold, air has escaped and the system raises an alarm. The threshold must be substantial enough to minimize false alarms. While the pressure drop may indicate a leak, the leak itself does not indicate whether the lost air went into the patient vasculature, into the body but outside the vasculature, or escaped from components that are external to the patient. Further, because system pressure is typically not in steady state during the counterpulsation cycle, the method may encounter significant false alarms.
- U.S. Pat. No. 5,242,374 entitled "Leak Detector for an Intra-Aortic Balloon Pump" filed on March 27, 1992 to Isoyama et al. describes leak detection by monitoring a position of the isolation diaphragm within the system.
- U.S. Pat. No. 4,522,194 entitled "Method and an Apparatus for Intra-Aortic Balloon Monitoring and Leak Detection" filed on June 10, 1983 to Normann describes leak detection by monitoring electrical impedance of the counterpulsation device.

Thus, past counterpulsation systems generally describe that the balloon should be fully inflated or slightly over-inflated during its inflation phase. Past systems viewed this inflation method as desirable to maximize counterpulsation support. Also, past counterpulsation systems have identified balloon leaks only after air has escaped from the balloon.

There is a significant need for a counterpulsation IABP device that is effective while only partially inflating the balloon to reduce balloon stress and inhibit air from escaping should a rift develop in the balloon's membrane.

### SUMMARY

A counterpulsation IABP device may avoid fully inflating the balloon to keep a damaged or defective balloon from releasing gas into the aorta while still providing effective cardiovascular circulation support. The device includes a balloon with a fully-inflated volume that is greater than an effective counterpulsation volume for the patient. For example, a device for a patient who needs the support afforded by a fully inflated 50 cc balloon (i.e., an effective counterpulsation volume of 50 cc) may be configured with a balloon having a fully-inflated volume that is greater than 50 cc (e.g., 55 cc or larger). A drive unit for the device may employ an air mover to deliver the effective counterpulsation volume (e.g., 50 cc) into the over-sized balloon. In some embodiments, the drive unit may employ an air mover such as a bellows, a compressed gas cylinder, a compressor, a pneumatic piston, a diaphragm, or other mechanism to deliver the effective counterpulsation volume into the over-sized balloon. In all embodiments, the over-sized balloon is under-inflated. Balloon under-inflation provides effective cardiovascular circulation support while avoiding stress on the balloon and preventing gas leakage in the event that high cycle fatigue damages the balloon wall.

A system for limiting inflation of an expandable member (e.g., a balloon) in an intravascular circulatory support system of a patient may comprise a drive unit and an air mover limiter. The drive unit may include an air mover and a motor. The air mover may have a first end and a second end. The first end may be fixed and have a pneumatic output in fluid connection with the expandable member. The second end may be movable and pneumatically closed. The air mover limiter may be configured to restrict displacement of the second end to under-inflate the expandable member based on the blood pressure of the patient. Displacement of the second end moves a volume of air into or out of the expandable member. The volume of air corresponds to the air mover limiter configuration.

### BRIEF DESCRIPTION OF THE DRAWINGS

Many aspects of the present technology can be better understood with reference to the following drawings. The components in the drawings are not necessarily to scale, and instead emphasis is placed on illustrating clearly the principles of the present disclosure.
FIG. 1 illustrates an example of an intravascular circulatory support system implanted within a patient's vasculature.
FIG. 2A illustrates a top perspective external view of an exemplary first embodiment of a drive unit for an intravascular circulatory support system.
FIG. 2B illustrates a top external view of the drive unit of FIG. 2A.
FIGs. 2C-2D illustrate an exemplary mechanical restriction for an air mover limiter.
FIG. 3 illustrates a top external perspective view of an exemplary second embodiment of a drive unit for an intravascular circulatory support system.
FIG. 4 illustrates a block diagram of certain control components applicable to the drive units of FIGs. 2A and 3.
FIG. 5 illustrates a flowchart of an exemplary method for an air mover limiter.

### DETAILED DESCRIPTION

Specific details of several embodiments of the technology are described below with reference to the figures. Although many of the embodiments are described below with respect to use of intravascular circulatory support systems/intravascular ventricular assist devices ("iVAD") that position an IABP/balloon in the aorta to provide counterpulsation that helps move blood through the body while preventing leaks due to balloon failure, other applications and other embodiments in addition to those described herein are within the scope of the technology. For example, several other embodiments of the technology can have different configurations, components, or procedures than those described herein, and features of the embodiments shown can be combined with one another. A person of ordinary skill in the art, therefore, will accordingly understand that the technology can have other embodiments with additional elements, or the technology can have other embodiments without several of the features depicted and described below.

The terminology used in the description presented below is intended to be interpreted in its broadest reasonable manner, even though it is being used in conjunction with a detailed description of certain specific embodiments of the present technology. Certain terms may even be emphasized below; however, any terminology intended to be interpreted in any restricted manner will be overtly and specifically defined as such in this Detailed Description section. Additionally, the present technology can include other embodiments that are within the scope of the examples but are not described in detail with respect to the figures.

Reference throughout this specification to "one embodiment" or "an embodiment" means that a particular feature, structure, or characteristic described in connection with the embodiment is included in at least one embodiment of the present technology. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

### Heart Failure and Circulatory Support Systems

Heart failure occurs when the heart is unable to maintain blood flow to meet the body's needs. This can occur if the heart cannot pump or fill adequately during contraction and relaxation, respectively. Heart failure is a common, costly, and potentially fatal condition. For example, heart failure currently affects about 6.5 million patients in the United States and is expected to increase by 46% by 2030. The stage/severity of heart failure can be defined using New York Heart Association (NYHA) classes, with NYHA Class I representing an early stage disease and NYHA Class IV representing a late stage disease. Current treatment options for heart failure depend on the stage of heart failure, and include, among other options, pharmacological therapy, cardiac resynchronization therapy (CRT), long-term mechanical circulatory support (e.g., left ventricular assist devices, or "LVAD"), and heart transplantation. Pharmacological therapy and CRT are typically used in relatively early stage cases (e.g., in patients with NYHA Class II or early NYHA Class III heart failure). However, these therapies typically only delay the progression of heart failure, meaning that even patients who initially respond to pharmacological therapy or CRT typically experience disease progression and required more advanced therapeutic interventions.

Prognosis for patients with heart failure remains poor: one-year mortality is about 15.0% for NYHA Class III patients and about 28.0% for NYHA Class 4 patients. This is at least partially due to the limited number of treatment options available for patients with late NYHA Class III/early NYHA Class IV heart failure. For example, while heart transplantation offers the best opportunity for long-term survival in late NYHA Class IV patients, this option is limited due to the scarcity of donor organs (e.g., approximately 2000 per year in the US, 200 per year in Canada, and less than 100 per year in Japan). Accordingly, many NYHA Class III and Class IV patients must rely on other treatments. For example, some patients receive LVAD therapy as a bridge to heart transplant or as a standalone therapy. However, LVAD therapy has several inherent shortcomings that limit their widespread use. Current LVAD therapies are expensive (e.g., over $100,000), typically require a major surgical procedure to implant (typically a sternotomy or thoracotomy), typically require use of cardiopulmonary bypass during the implant procedure, and typically require blood products (e.g., about 11.6 units of blood products). LVAD therapies that are implanted through less-invasive means (e.g., percutaneously) are only used for short-term circulatory support. Furthermore, postoperative care of patients who receive a LVAD can be challenging and costly, and patient anxiety can be high because the devices cannot be shut off or lose power for more than a few minutes. LVAD therapy is also associated with several serious adverse events such as device failure, thrombosis, thromboembolism, stroke, infection, and bleeding. For at least these reasons, LVAD therapy is typically reserved for patients with end-stage heart failure who have limited options (e.g., late NYHA Class IV). This leaves a large percentage of heart failure patients who have cases that are too advanced for CRT but are not yet severe enough to justify LVAD therapy/heart transplantation (e.g., late NYHA Class III/early NYHA Class IV patients) without effective treatment options. There are currently about 1.6 million patients in the United States and about 3.9 million patients in Europe with late Class III/ early Class IV heart failure, representing a large patient population with limited treatment options.

Another treatment option for heart failure is counterpulsation therapy using an intraaortic balloon pump (IABP). Counterpulsation therapy is achieved by rapidly inflating a balloon positioned in the patient's aorta immediately after aortic valve closure (dicrotic notch) and rapidly deflating the balloon just before the onset of systole. The rapid inflation of the balloon increases the diastolic aortic pressure by 25-70%, augmenting end-organ and coronary perfusion. The rapid deflation of the balloon reduces the ejection pressure of the native ventricle, reducing afterload and left ventricular external work.

Counterpulsation therapy is an attractive therapy option because using an IABP is much simpler than implanting and using an LVAD and is associated with fewer adverse events. For example, a physician can implant an IABP without directly cannulating the heart. However, conventional counterpulsation systems implanted through minimally invasive procedures can only be used for short durations. This is this case for several reasons. For example, the arterial access (e.g., the femoral artery), the durability of the IABP, generally, and the balloon in particular, and biocompatibility issues can limit the use of IABP to short durations of less than about 14 days. Longer duration of IABP support (>14 days) can lead to an increase in the frequency of vascular complications, infections, balloon rupture, and bleeding. Moreover, in its current form, a catheter mounted IABP advanced retrograde from the femoral artery into the descending aorta requires the patient to remain supine for the duration of therapy. Consequently, patients cannot be ambulatory or be discharged from the hospital. These limitations prevent the IABP from being used as an extended therapy for heart failure and instead are used in short term settings, such as in patients awaiting transplant and in patients undergoing coronary artery bypass surgery.

The assignee/applicant (NuPulseCV, Inc.) has developed various counterpulsation support systems designed to provide longer-term support to patients suffering from heart failure as compared to the above-described conventional systems. Such improved counterpulsation support systems are described in U.S. Patent No. 7,892,162 entitled "ARTERIAL INTERFACE" filed October 22, 2009, U.S. Patent No. 8,066,628 entitled "INTRA-AORTIC BALLOON PUMP AND DRIVER" filed October 22, 2010, U.S. Patent Application Serial Number 15/685,553 entitled "BLOOD PUMP ASSEMBLY AND METHOD OF USE THEREOF" filed August 24, 2017, U.S. Patent Application Serial Number 16/876,110 entitled "INTRAVASCULARLY DELIVERED BLOOD PUMPS AND ASSOCIATED DEVICES, SYSTEMS, AND METHODS" filed on May 17, 2020, U.S. Patent Application Serial Number 17/944,130 entitled "INTRA-AORTIC BALLOON PUMP ASSEMBLY WITH PRESSURE SENSOR" filed on September 13, 2022, U.S. Patent Application Serial Number 17/944,125 entitled "BLOOD PUMP SUPPORT APPARATUS AND METHOD FOR A BLOOD PUMP ASSEMBLY" filed on September 13, 2022, U.S. Patent Application Serial Number 17/944,127 entitled "INTRA-AORTIC BALLOON PUMP ASSEMBLY" filed on September 13, 2022, and U.S. Patent Application Serial Number 17/878,632 entitled "DRIVE UNIT FOR INTRAVASCULAR CIRCULATORY SUPPORT SYSTEMS" filed on August 1, 2022, the entire disclosures of each of the foregoing is incorporated by reference herein.

The IABPs described in the incorporated disclosures offer long-term or chronic counterpulsation therapy for heart failure patients with components that, at least in certain instances, can be implanted using minimally invasive percutaneous procedures. For example, the IABP can be implanted minimally invasively without entering the chest, and generally do not require cardiopulmonary bypass or administration of blood products.

The present technology offers a drive unit that can direct a fluid (gas or liquid, e.g., air) into an internal volume of a balloon or expandable member (e.g., the IABPs described in the above-incorporated disclosures) that has been implanted into the patient's vasculature to provide effective cardiovascular circulation support while avoiding stress on the balloon and preventing gas leakage in the event of balloon rupture. Drive unit hardware and/or software controls expandable member inflation/deflation cycles to prevent full inflation. This under-inflation avoids the formation of rifts in the expandable member due to high inflation/deflation cycle fatigue. Furthermore, should a rift form in the expandable member membrane, this under-inflation will not permit gas to escape into the patient vasculature.

### Select Embodiments of Chronic Intravascular Circulatory Support Systems

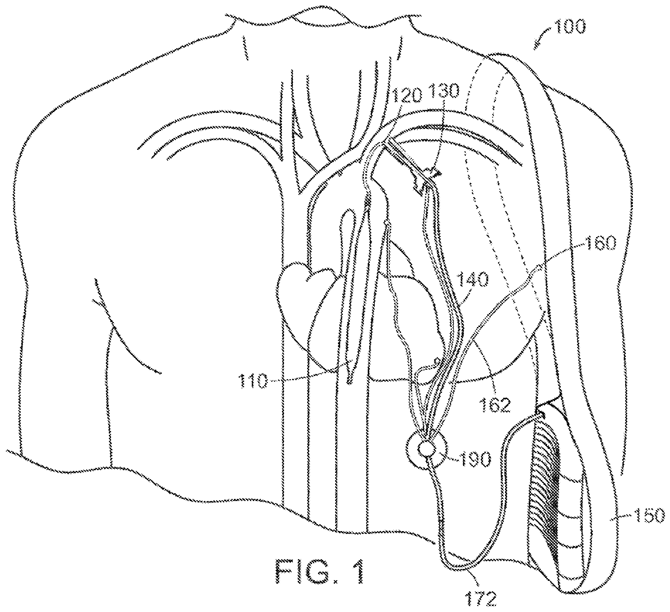

FIG. 1 illustrates a circulatory support and/or intravascular ventricular assist system 100 configured in accordance with select embodiments of the present technology. The system 100 can include an expandable member 110 implantable in an aorta of a patient suffering from heart failure. Expandable member 110 may be referred to as an IABP or balloon. The system 100 can further include a first pneumatic driveline 120 (also referred to as an "internal driveline"), an arterial interface device or stopper 130, a second pneumatic driveline 140, a skin interface device 190, a drive unit 150, and sensors 160. When implanted, the system 100 can provide counterpulsation therapy to a patient suffering from heart failure.

The expandable member 110 can be a balloon or other element that can change size and/or shape in response to being filled with a gas or liquid. For example, in some embodiments the expandable member 110 is a balloon composed of a biocompatible, non-thrombogenic elastomeric material (e.g., Biospan^{®}-S). The expandable member 110 can also be made of other suitable materials. The expandable member 110 is transitionable between at least a first state in which it is generally deflated and a second state in which it is generally inflated to an effective counterpulsation volume.

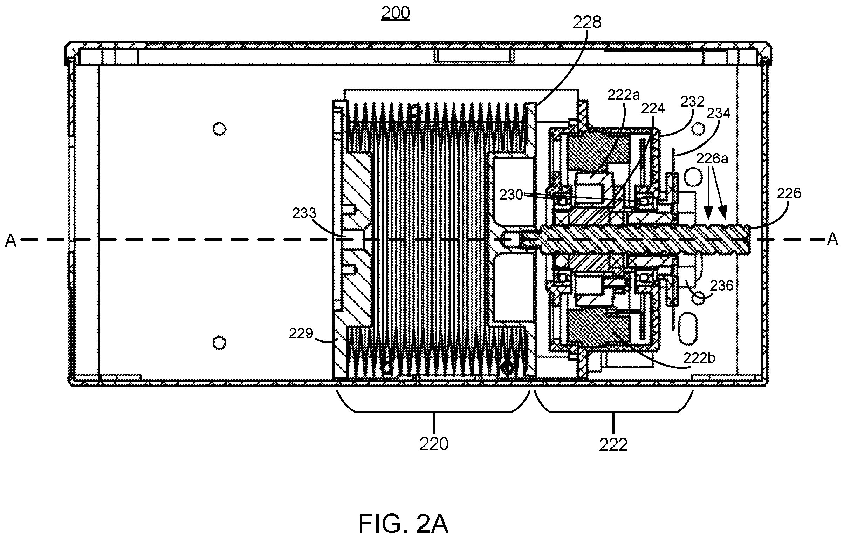

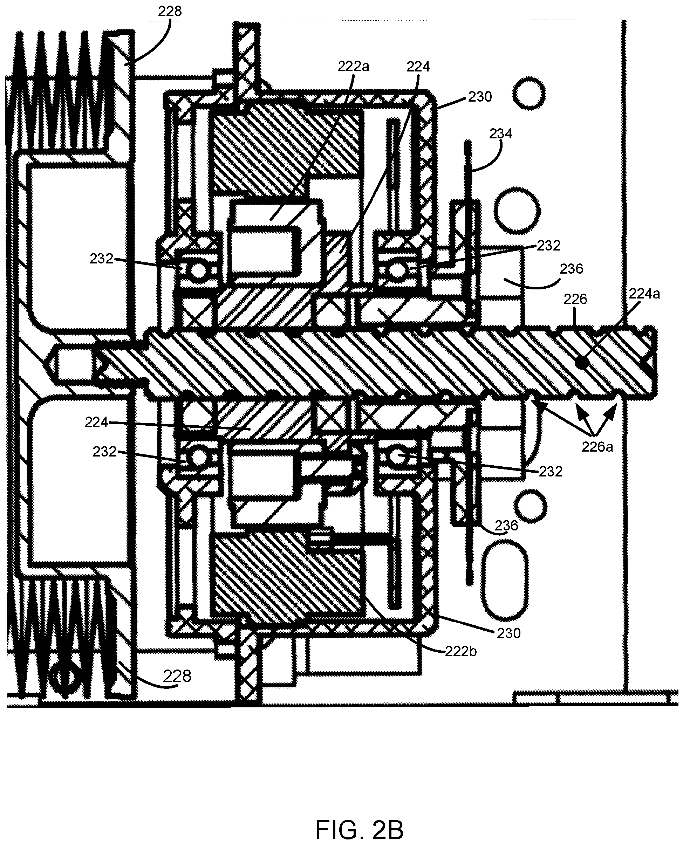

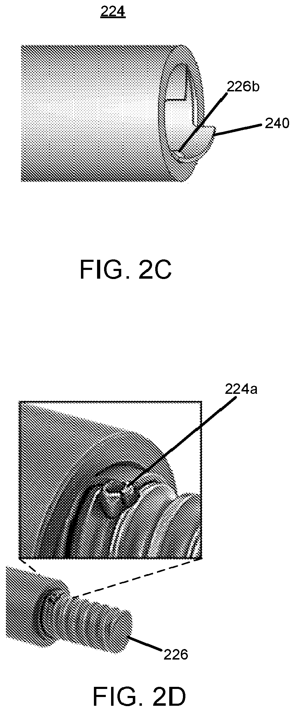

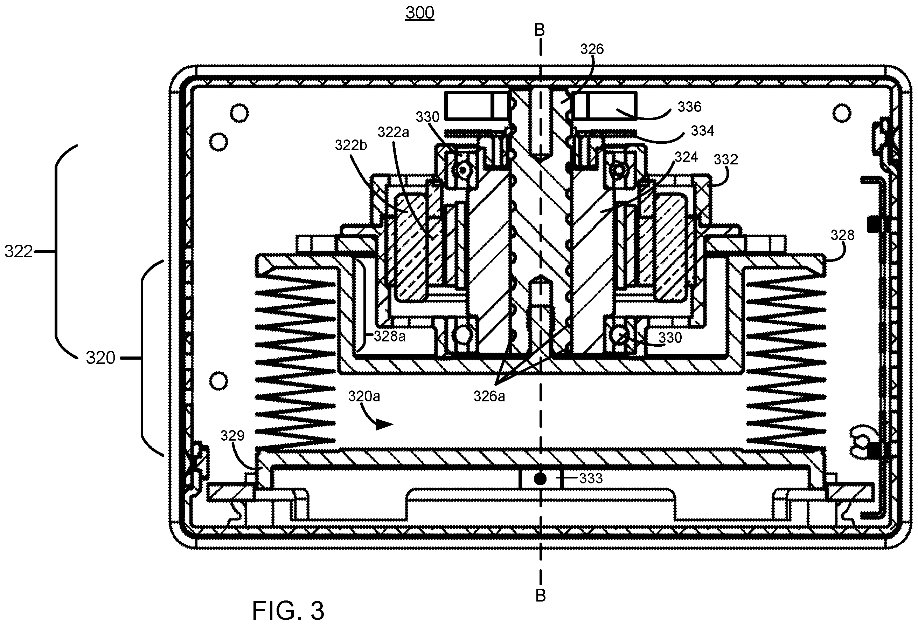

An effective counterpulsation volume may be a volume of fluid that occupies the expandable member 110 after rapidly inflating the expandable member 110 positioned in the patient's aorta immediately after aortic valve closure (dicrotic notch). The effective counterpulsation volume within the expandable member 110 increases the diastolic aortic pressure by 25-70%, augmenting end-organ and coronary perfusion. The effective counterpulsation volume may be based on a lowest therapeutic diastolic blood pressure for a particular patient, or on an average lowest therapeutic diastolic blood pressure for a patient population. The size of the expandable member 110 and/or an air mover 220, 320 of the drive unit 200, 300 (FIGs. 2 and 3) is selected so that the effective counterpulsation volume is less than the fully inflated volume of the expandable member 110. For example, an effective counterpulsation volume for a patient may be 50 cc to achieve the lowest therapeutic diastolic blood pressure for the patient, or the average lowest therapeutic diastolic blood pressure for a patient population. To prevent undue inflation stress on the expandable member 110 and prevent gas from leaking from a ruptured expandable member in the patient's vasculature, the expandable member 110 may include a fully inflated volume of greater than 50 cc (e.g., 55 cc), while the air mover 220, 320 may be configured to expel a volume of air that is less than the fully inflated volume of the expandable member 110 (i.e., the lowest therapeutic diastolic blood pressure for the patient, or the average lowest therapeutic diastolic blood pressure for a patient population).The "air mover" 220, 320 may include a bellows, a compressed gas cylinder, a pneumatic piston, a diaphragm, a compressor, or other mechanism to deliver the effective counterpulsation volume into the over-sized balloon. The bellows includes a pneumatic output in fluid connection with the expandable member 110, a first end (e.g., the static flange 229, 329 of FIGs. 2A, 2B, and 3) and a second end (e.g., the dynamic flange 228, 328 of FIGs. 2A, 2B, and 3). The first end is fixed and the second end is displaceable. While the embodiments of FIGs. 2A, 2B and 3 describe the "air mover" as a bellows 220, 320, the "air mover" is not limited to the bellows configuration and may also include any mechanism to deliver the appropriate gas volume into the expandable member 110.

Full inflation of the expandable member 110 depends on various parameters of the system 100. For example, the expandable member 110 being "fully inflated" may depend on pneumatic system volume, a size or capacity of the expandable member 110, changes in volume of the bellows 220, 320 corresponding to inflation and deflation cycles, and patient blood pressure. With the exception of patient blood pressure, these parameters may be measured or compiled from the system 100 and its components as described in relation to FIG. 1. The blood pressure threshold below which a leak could occur is, therefore, the lowest therapeutic diastolic blood pressure for the patient. Accounting for the volumes of the pneumatic system, expandable member, and the low-end threshold of blood pressure, movement of the bellows 220, 320 is limited to prevent full inflation of the expandable member 110. As described herein, the limitation on the bellows movement may be implemented mechanically or by software instructions.

Accordingly, the expandable member 110 can provide counterpulsation therapy without risk of expandable member 110 rupture or fluid leak into the patient's vasculature by repeatedly transitioning between the first state (deflation) and the second state (inflation) without exceeding an effective counterpulsation volume. To transition the expandable member 110 between the first state and the second state, the drive unit 150 can direct the effective counterpulsation volume of fluid (gas or liquid, e.g., air) into an internal volume of the expandable member 110 via the first pneumatic driveline 120 and the second pneumatic driveline 140, as described in greater detail below. The expandable member 110 can also be sized and/or shaped to reduce and/or prevent the expandable member 110 from blocking arteries branching from the aorta, such as the renal arteries, and to have an internal volume when fully inflated that is less than the effective counterpulsation volume. In some embodiments, the expandable member 110 may also have certain features generally similar to those described in disclosures incorporated herein by reference. In some embodiments, the expandable member 110 includes an expandable end effector other than or in addition to a balloon.

The drive unit 150 can generate gas flow into and out of the expandable member 110 via the first pneumatic driveline 120 and the second pneumatic driveline 140 that converge to form an external pneumatic driveline 172 extending from the drive unit 150. For example, the drive unit 150 can generate a positive pressure to accelerate gases into the expandable member 110 via the first pneumatic driveline 120 and the second pneumatic driveline 140, thereby inflating the expandable member 110. The drive unit 150 can also induce a negative pressure to withdraw gases from the expandable member 110 via the first pneumatic driveline 120 and the second pneumatic driveline 140, thereby deflating the expandable member 110. The drive unit 150 can induce gas flow into and out of the expandable member 110 through a bellows (not depicted in Fig. 1). In some embodiments, the drive unit 150 can control the volume of air being pushed into the expandable member 110 to avoid fully inflating or overinflating the expandable member 110. For example, in an embodiment utilizing a bellows to generate air flow, the volume of airflow generated by the bellows (e.g., the volume of the bellows) can correspond to the lowest therapeutic diastolic blood pressure for the patient, or the average lowest therapeutic diastolic blood pressure for a patient population that is less than an interior volume of the expandable member 110. In other embodiments, the volume of fluid expelled by the bellows may be restricted by an air mover limiter (not depicted in Fig. 1) to reduce the capacity of the bellows to correspond to an effective counterpulsation volume that is also less than an interior volume of the expandable member 110. The air mover limiter may be mechanical, software instructions controlling mechanical components, or a combination of mechanical and software components. One of skill in the art will recognize that the volume of the air mover may be different than the interior volume of the balloon due to changes in relative pressure. In some embodiments, the drive unit 150 uses ambient air from the environment surrounding the drive unit 150 (e.g., "room air") to drive operation of the system 100. Using ambient air is expected to reduce the size, weight, and/or cost of the drive unit 150 relative to a drive unit that relies on an internal gas or fluid supply (e.g., helium tanks). For example, in some embodiments the drive unit 150 can weigh about 3 kg or less. The driveline can be disconnected near the skin interface device 190 when the system 100 is not being actively used.

The system 100 may allow chronic support of heart function and blood flow, while still allowing the patient to remain ambulatory. Typical ventricular assist devices require femoral access for the driveline and/or connection to large, stationary external control units, and therefore confine the patient to a hospital bed in the supine position for the duration of therapy. In contrast, the system 100 allows the patient to move about relatively unencumbered. Moreover, the therapy level provided by the system 100 can be adjusted to match patient need. For example, the effective counterpulsation volume and support ratio (e.g., 1:1, 1:2, 1:3 support) provided by the expandable member 110 can be adjusted for each patient to vary the support provided while still underinflating the expandable member 110 to prevent undue stress and/or gas leakage. One of skill in the art will recognize that the support ratio measures the ratio of beats to inflations of the balloon. For example a 1:1 support ratio indicates the for every beat there is a corresponding inflation of the balloon, whereas a 1:2 support ratio indicates that there are two beats before each inflation, and a 1:3 support ratio indicates that there are three beats before each inflation.

The drive unit 150 can have a user interface (not depicted) where a clinician can control the effective counterpulsation volume or support ratio. Alternatively, drive unit 150 can be controlled via other external inputs (e.g., using a corresponding tablet or other device that is in electrical communication with drive unit 150). Gradually reducing the effective counterpulsation volume over time (e.g., by changing the desired travel of an internal bellows mechanism as a percent of the full travel) can result in a controlled loading of the heart which in some instances may be beneficial for cardiac recovery. Likewise, the travel of the internal bellows mechanism may be restricted mechanically and/or by computer instruction via the user interface to limit the inflated volume of the expandable member 110 to below the fully-inflated volume. Furthermore, unlike conventional circulatory support systems, the system 100 can be turned off to, for example, assess the ability of the patient to handle cardiac demand without support before removing the system 100, or for another suitable reason. In some embodiments, for example, the expandable member 110 is designed to remain in the aorta in a deflated condition for a relatively prolonged duration (e.g., for 23 hours in a single day). This is in direct contrast to conventional devices, which often must be removed if turned off for more than about fifteen minutes because reactivation could result in a shower of emboli that formed when in the inactive state.

### Drive Unit

The present disclosure contemplates at least two embodiments of drive unit 150. The first embodiment is depicted in FIGs. 2A-B and refers to the drive unit by reference numeral 200. The second embodiment is depicted in FIG. 3 and refers to the drive unit by reference numbers 300.

FIG. 2A depicts a first embodiment of drive unit 200. The drive unit 200 may include a bellows 220 and a motor 222 (e.g., an electric motor such as a brushless DC motor. The space to the left of bellows 220 in FIG. 2A may be at least partially occupied by electrical components (not illustrated) used to control motor 222. Such components may include one or more computer processors and memory containing computer processor readable instructions capable of being executed by the computer processor. The same space may be at least partially occupied by mechanical components such as value manifolds (e.g., for coupling bellows output/pneumatic output 223 to an applicable port 204).

Bellows 220 may be an axial expansion bellows that is able to expand and contract along the A-A axis depicted in Fig. 2A in response to rotation of the motor 222 in different directions. In the embodiment depicted by FIGs. 2A and 2B, the bellows 220 is expanded such that the expandable member 110 (FIG. 1) is deflated. When the bellows 220 is compressed by motor 222, the expandable member 110 (FIG. 1) is inflated. The bellows 220 may have a constant cross-sectional geometry along its length so that the volume within the bellows 220 is varied according to the bellows length.

The motor 222 may include a rotor 222a and stator 222b. The rotor 222a may be joined to a rotary-to-linear transformer. For example, the rotor 222a may be joined to a ball nut 224 carrying a ball screw 226 that is affixed to a dynamic flange 228. The bellows 220 may be sealed by the dynamic flange 228 at a bellows second end proximal to the ball screw 226 and by a static flange 229 (FIG. 2A) at a bellows first end that is distal to the ball screw 226. A bellows outlet 233 (FIG. 2A) may allow communication of fluid within the external drive line 172 to the expandable member 110 in response to movement or displacement of the bellows second end (i.e., the dynamic flange 228).

In some embodiments, the bellows outlet 233 may be formed within the static flange 229. The ball screw 226 in combination with the ball nut 224 may form a mechanical rotary-to-linear transformer that converts rotational motion of the motor 222 to linear motion with little friction. Rotation of the ball nut 224 by the rotor 222a within the stationary stator 222b may cause the ball screw 226 to move linearly along axis A-A and, correspondingly, cause linear movement of the bellows 220.

The ball screw 226 may be threaded to provide a helical raceway 226a for balls 226b (FIG. 2C) of the ball nut 224 and may act as a precision screw. Several dimensions may define the ball screw 226 and raceway 226a. For example, the ball screw 226 may include a pitch measuring the distance between grooves of the helical raceway 226a.

The rotor 222a and ball nut 224 assembly may be mounted to a housing 230 of the motor 222 via radial bearings 232. The inner race of the radial bearings 232 may be affixed to the rotor 222a and ball nut 224 assembly while the outer race of the radial bearings 232 and the stator 222b may be affixed to a motor housing 230. Actuation of the motor 222 may cause rotational movement of the rotor 222a and ball nut 224 which may cause balls 226c (FIG. 2C) of the ball nut 224 to ride within the helical raceway 226a of the ball screw 226 and convert the rotational movement of the rotor 222a into linear movement of the ball screw 226 along axis A-A (FIG. 2A).

Movement of the ball screw 226 is translated to the bellows 220 via the dynamic flange 228, causing the bellows 220 to expand or contract to create negative or positive fluid flow, respectively, within the external drive line 172, which is in fluid connection with the expandable member 110 via the bellows outlet 233 (FIG. 2A). In some embodiments, the ball screw 226 may be fixedly mated to the dynamic flange 228 via a threaded interface (as depicted in FIG. 2B). In some embodiments, the dynamic flange 228 and ball screw 228 are one continuous piece. When the ball screw 226 is fixedly mated to the dynamic flange 228 or when the ball screw 226 and dynamic flange 228 are one continuous piece, ball screw 228 may directly and efficiently translate its motion to the bellows (e.g., withdrawal of the ball screw 226 away from the static flange 229 may pull the dynamic flange 228 to expand the bellows 220).

With reference to FIGs. 2C-2D, in some embodiments, the ball nut 224 may be configured as an air mover limiter to mechanically restrict contraction of the bellows and positive fluid flow within the external drive line 172 such that the expandable member 110 is inflated to an effective counterpulsation volume, but remains under-inflated. In some embodiments, the air mover limiter may be a ball nut 224 including a stop pin 224a. The air mover limiter may allow the ball nut 224 to spin while limiting travel of the bellows 220 along the ball screw 226 via the dynamic flange 228 by stopping further linear movement of the air mover limiter 224 along the axis A-A. A clutch-like collar 240 inside the air mover limiter 224 prevents the air mover limiter 224 from travelling past the stop pin(s) 224a. The stop pin 224a may be placed in a valley or root of the raceway 226a (e.g., by drilling a hole and pressing in the stop pin). The stop pin(s) 224a are placed to limit the ball nut 224 from spinning along the ball screw 226 to under-inflate the expandable member 110. When the collar 240 meets the stop pin 224a, the air mover limiter 224 disengages from the raceway and begins to idle. To start the air mover limiter 224 travelling again, spin the rotor 222a and air mover limiter 224 assembly in the opposite direction. The air mover limiter may include other mechanical means to limit the travel of the bellows 220 or to otherwise restrict inflation of the expandable member to less than a fully-inflated volume.

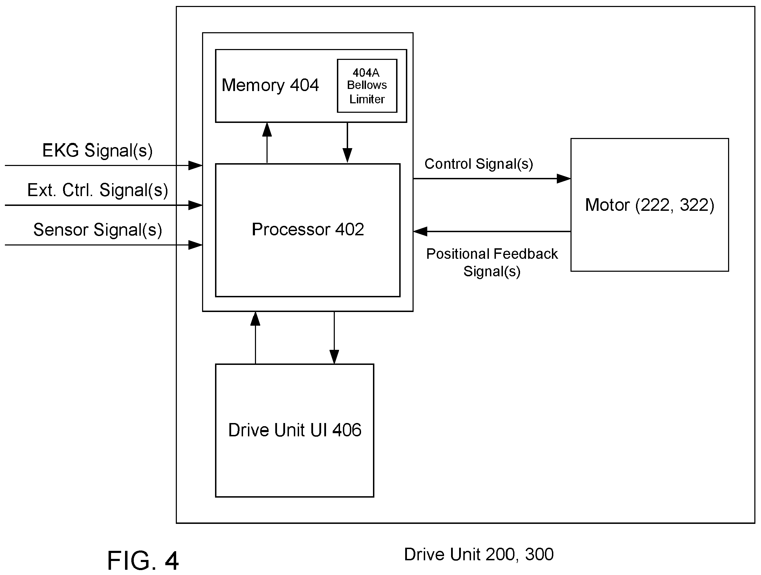

With reference to FIGs. 2A-B, and 4, drive unit 200 may include processor 402, memory 404, and drive unit 402. Processor 402 may include one or more dedicated or non-dedicated micro-processors, micro-controllers, sequencers, micro-sequencers, digital signal processors, processing engines, hardware accelerators, applications specific circuits (ASICs), state machines, programmable logic arrays, any integrated circuit(s), discrete circuit(s), etc. that is/are capable of processing data or information, or any suitable combination(s) thereof. Memory 404 may include any suitable non-volatile memory device, chip, or storage device capable such as one or more of: system memory, frame buffer memory, flash memory, random access memory (RAM), read only memory (ROM), a register, and a latch. Processor 402 is capable of executing executable instructions (e.g., as stored in memory 404). Processor 402 may be configured to control motor 222, and by extension, the bellows 220.

For example, an encoder disk 234 and encoder sensor 236 may determine the angular position, speed, and/or direction of the rotor and provide such information as positional feedback signal(s) to a processor 402 and/or memory 404. The encoder disk 234 is a component of an encoder which includes the encoder sensor 236. In combination with the encoder sensor 236, the encoder disk 234 converts mechanical motion into electrical signals. Encoder disks are typically made of metal, plastic, glass, or paper, and they have a series of segments. The encoder sensor 236 is able to differentiate between the segments to determine disk rotation. In some embodiments, the segments are clear and opaque, magnetic and non-magnetic, white and black, or any other series of alternating materials or appearances. Rotation of the disk 234 through the sensor 236 creates a series of electrical pulses that can be used to measure the speed, position, or direction of the encoder disk's rotation and, thus, the motor 222 rotation.

The encoder disk 234 may also include a "home" position. The home position of an encoder disk is the position where the encoder is pointing to the "zero value" or default position of the encoder when it is turned on or reset. Incremental encoder disks determine the home position by detecting the start of a pattern on the disk. For example, the disk 234 might have a pattern of alternating black and white lines. The encoder would start counting when it detects the first black line. Absolute encoder disks may determine the home position by reading a code value on the disk. For example, the disk might have a code that represents the number 0. Processor 402 and/or memory 404 (FIG. 4) may receive the code from the sensor 236, 336 (FIGs. 2 and 3) and execute instructions to store the code in memory 404. The encoder sensor 236 may read the code and then set the encoder disk 234 position to 0. The home position can be important for a variety of reasons, such as measuring rotation of the ball screw 228 and translation of that movement into a volume of gas within or expelled by the bellows 220, or alignment of the encoder with another device. Once the home position has been set, the processor 402 may execute instructions to return the encoder disk 234 to that position when it is turned off or reset. This ensures that the encoder is always in the correct position.

Alternatively, a linear position sensor (not depicted) may be used to determine the position of the ball screw 226 or bellows 220 and to provide such positional feedback signal(s). Processor 402 and/or memory 404 may also receive control information from drive unit UI 406 and provide display information (e.g., status information) to drive unit UI 406. Relatedly, processor 402 and/or memory 404 may similarly receive EKG signal(s) from skin interface device 190 and one or more sensors 160 (FIG. 1), one or more external control signals (e.g., from a tablet or other computing device (not depicted)), and one or more sensor signals. For example, processor 402 and/or memory 404 may receive a pressure signal as observed by a pressure sensor (not depicted) located in proximity to balloon 110 when disposed in an artery (e.g., the descending aorta, as depicted in FIG. 1) of a patient undergoing therapy (e.g., counterpulsation therapy). The one or more pressure signals may be, indicative of the pressure exerted on balloon 110 in such artery and/or the pressure within such artery.

Processor 402 may use one or more of the positional feedback signals, drive unit UI 406 control signals, EKG signals, external control signals, and sensor signals to control motor 222. For example, EKG signals may be used to ensure proper timing of the inflation and/or deflation of the balloon 110 (e.g., to pursue counterpulsation). And drive unit UI 406 control signal and external control signals may be used to change the effective counterpulsation volume, support ratio, and maximum inflation volume as discussed *supra.*

The various signals described above could be monitored to determine when the patient's blood pressure falls below the lowest therapeutic diastolic blood pressure for the patient, or the average lowest therapeutic diastolic blood pressure for a patient population. This determination would signal full or over-inflation of the expandable member 110 and be used to further adjust the bellows travel to below the previously-determined volume to keep the expandable member under-inflated. The contour of the bellows pressure during inflation dwell or the patient's blood pressure during the partial cycle, or some combination of these measurements may also be used to signal full or over-inflation.

Furthermore, under-inflation of the expandable member 110 may be useful to detect ruptures in the expandable member 110. For example, because the pressure from under-inflation is never higher than the lowest diastolic pressure, blood may enter the pneumatic system through a crack or rift in the expandable member 110 that may develop due to high cycle fatigue and grow steadily in size over time with continued use. Blood escaping through the crack or rift becomes visible in the pneumatic driveline. Under-inflation allows blood to ingress, but air cannot egress when the rift is small. Observation of blood in the driveline will signal the need to discontinue support.

Other embodiments may employ linear brushless DC motors, solenoids, and/or piezo electric actuators to compress and expand bellows 220.

Drive unit 200 may be used to effectively inflate and deflate the inflatable member 110 using ambient air to provide counterpulsation therapy in patients with heart failure. The following component values set forth in the table below are exemplary for such purposes for counterpulsation using the drive unit 200 and an approximately 20-60cc balloon as the inflatable member 110.

| Drive Unit 200 Component (units) | Dimensions |
|---|---|
| Bellows Outside Diameter (mm) | 90 |
| Bellows Piston Area (cm²) | 48.2 |
| Bellows Travel (mm) | 17.7 |
| Drive Unit Height (mm) | 87 |
| Envelope Volume / Size of Bellows Plus Drivetrain (cc) | 557 |

Although effective for counterpulsation in general, drive unit 200 as configured above, uses a significant amount of power merely to overcome the rotational inertia of rotor 222a and may not be "fast enough" to treat patients with heart arrhythmias, irregular heartbeats, or a-fib. Ball screws pitch may be selected or adjusted to improve efficiency of the drive unit. In particular, adjusting the ball screw pitch (and/or adjusting the diameter of the bellows 220) may better match the impedance of the motor 222 and the impedance of the pneumatic load on the drive unit 200 (e.g., the collectively pneumatic load of balloon 110, first pneumatic driveline 120, second pneumatic driveline140, and external driveline 172). Such an improvement in efficiency may improve the battery life of any battery employed to drive motor 222.

With reference to FIG. 3, a second embodiment of the drive unit 300 may include a bellows 320 and a motor 322 (e.g., an electric motor such as a brushless DC motor). Bellows 320 may be an axial expansion bellows that is able to expand and contract along the B-B axis depicted in Fig. 3 in response to rotation of the motor 322 in different directions. As depicted in Fig. 3, the bellows 320 may include a dynamic flange 328. The dynamic flange 328 may be sized and shaped to receive or house at least a portion of the motor 322 such that at least a portion of the motor 322 is nested within a bellows cavity 320a. For example, the direction of the recess 328a may be toward the bellows cavity 320 and take a three-dimensional shape (e.g., a cylinder) suitable for receiving or housing and nesting at least a portion of the motor 322 within the bellows cavity 321a while maintaining sufficient stroke length to provide effective air flow within the external drive line 172. The recess 328a may allow a reduction in size and weight of the outer case 302, improving mobility of the system (e.g., as compared to the size of outer case 202). In the embodiment depicted in FIG. 3, the bellows 320 is depicted in an expanded or uncompressed state such that the expandable member 110 (FIG. 1) is deflated.

The motor 322 includes a rotor 322a and stator 322b. As with the first embodiment 200, the rotor 322a may be shaped to join a rotary-to-linear transformer, for example, a ball nut 324 carrying a ball screw 326 that is affixed to a dynamic flange 328 via a threaded interface. In other embodiments, the rotor 323 and the ball nut 324 may be formed as a single piece. In some embodiments, the ball screw 326 is hollow to reduce weight. The rotor 323 and ball nut 324 assembly may be mounted to a housing of the motor 332 via radial bearings 330. The inner race of the radial bearings 330 may be affixed to the rotor 323 and ball nut 324 assembly while the outer race of the radial bearings 330 and the stator 323b may be affixed to a motor housing 332. Like the first embodiment, actuation of the motor 322 may cause rotational movement of the rotor 323 and ball nut 324 which may translate into linear movement of the ball screw 326 along axis B-B. Movement of the ball screw 326 may be translated to the bellows 320 via the dynamic flange 328, causing the bellows 320 to expand or contract to create negative or positive fluid flow, respectively, within the external drive line 172, which is in fluid connection with the expandable member 110 via a bellows output/pneumatic output 333 associated with the static flange 329. Other embodiments may employ linear brushless DC motors, solenoids, and/or piezo electric actuators to compress and expand bellows 320.

The ball screw 326 may be mated to the dynamic flange 328 (or the two components may be one continuous piece) to allow the ball screw 328 to directly and efficiently translate its motion to the bellows 320 while the rotor 322a and ball nut 324 spin within the stator 322b around axis B-B. Rotation of the rotor 322a may carry the balls of the ball nut 324 within the helical raceway 226a of the ball screw 226, thus causing movement of the ball screw 226 along axis B-B. In some embodiments, the ball nut 324 may be configured as an air mover limiter 224, as described in relation to FIGs. 2C-2D to restrict movement or displacement of the dynamic flange 328 and positive fluid flow within the external drive line 172 such that the expandable member 110 is inflated to an effective counterpulsation volume, but remains under-inflated.

In reference to FIGs. 3 and 4, an encoder disk 334 and encoder sensor 336 may determine the angular position, speed, and/or direction of the rotor and provide such information to a processor via electrical feedback signals (not depicted). The encoder disk 334 is a component of an encoder which includes the encoder sensor 336 and includes the same advantages described above in relation to encoder disk 234 and encoder sensor 236. In other embodiments, a linear position sensor (not depicted) may be used to determine the position of the ball screw 326 or bellows 320 and to provide that information to such processor. Such a processor may also receive EKG signals from skin interface device 190 and one or more sensors 160 (FIG. 1), suitable external control signals (e.g., from a tablet (not depicted) configured to control motor 322 and the travel of bellows 220, a user interface operatively coupled to the case 302 (not depicted), etc.), and other sensors (e.g., pressure sensors (not depicted) capable of sensing the pressure in the descending aorta).

Drive unit 300 may be used to effectively inflate and deflate inflatable member 110 using ambient air to provide counterpulsation therapy in patients with heart failure, including patients with heart arrhythmias, irregular heartbeats, or a-fib. The following ranges of component values may be efficient and/or economical for counterpulsation using drive unit 300 and an approximately 20-60cc balloon as inflatable member 110.

| Drive Unit 300 Component (units) | Range of Dimensions | |
|---|---|---|
| Bellows Outside Diameter (mm) | 125 | 111 |
| Bellows Piston Area (cm²) | 92.3 | 73 |
| Bellows Travel (mm) | 8 | 11.5 |
| Drive Unit Height (mm) | 71 | 75 |
| Envelope Volume / Size of Bellows Plus Drivetrain (cc) | 865 | 726 |
| Ball Screw Pitch (mm) | 3.5 | 5.5 |
| Peak Motor Torque (N*m) | 0.22 | 0.19 |
| Plateau Speed (rpm) | 1380 | 1365 |
| Average Power (watts) | 5.37 | 5.1 |

With reference to FIG. 4 , drive unit 200, 300 may include processor 402, memory 404, and drive unit 402. Encoder disk 234, 334 and encoder sensor 236, 336 may determine the angular position, speed, and/or direction of the rotor and provide such information as positional feedback signal(s) to a processor 402 and/or memory 404. In other embodiments, a linear position sensor (not depicted) may be used to determine the position of the ball screw 226, 326 or bellows 220, 320 and to provide such positional feedback signal(s). Processor 402 and/or memory 404 may also receive control information from drive unit UI 406 and provide display information (e.g., status information) to drive unit UI 406. Relatedly, processor 402 and/or memory 404 may similarly receive EKG signal(s) from skin interface device 190 and one or more sensors 160 (FIG. 1), one or more external control signals (e.g., from a tablet or other computing device (not depicted)), and one or more sensor signals. For example, processor 402 and/or memory 404 may receive a pressure signal as observed by a pressure sensor (not depicted) located in proximity to balloon 110 when disposed in an artery (e.g., the descending aorta, as depicted in FIG. 1) of a patient undergoing therapy (e.g., counterpulsation therapy). The one or more pressure signals may be, indicative of the pressure exerted on balloon 110 in such artery and/or the pressure within such artery.

Processor 402 may use one or more of the positional feedback signals, drive unit UI 406 control signals, EKG signals, external control signals, and sensor signals to control motor. For example, EKG signals may be used to ensure proper timing of the inflation and/or deflation of the balloon 110 (e.g., to pursue counterpulsation). And drive unit UI 406 control signal and external control signals may be used to change the effective counterpulsation volume, support ratio, and maximum inflation volume, as discussed *supra.*

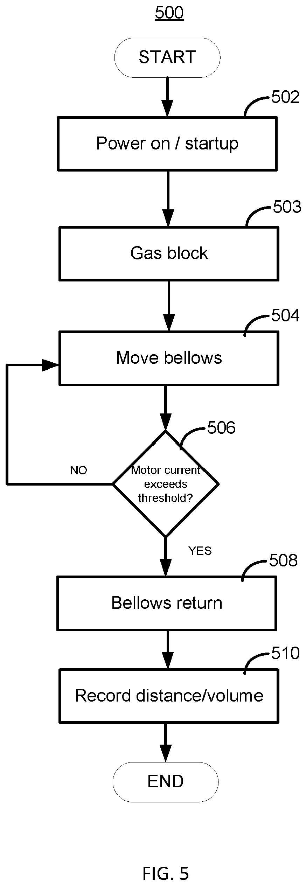

FIG. 5 is a flowchart of a software restriction including processor-executable instructions for method 500 for initializing the drive unit 200, 300 for counterpulsation support to achieve the lowest therapeutic diastolic blood pressure for the patient, or the average lowest therapeutic diastolic blood pressure for a patient population. Each step of the method 500 is one or more processor-executable instructions performed on a microprocessor or other computing device (e.g., drive unit 200, 300 including processor 402 and memory 404) which may be physically configured to execute the different aspects of the method. Each step may include execution of any of the instructions as described in relation to the drive unit 200, 300. While the below blocks are presented as an ordered set, the various steps described may be executed in any particular order to complete the air mover limiter methods described herein.

At block 502, the processor 402 may execute a "power on" or "startup" command. For example, processor-executable instructions stored in the memory 404 may cause the processor 402 to begin an initialization sequence for the drive unit 200, 300. At block 503, the processor may execute a "gas block" command so that no gas is pushed into the bellows 220, 320 during execution of the method 500. For example, block 503 may include the processor executing a command to configure (i.e., open or close) one or more valves in the system 100 so that no gas is pushed into the bellows 220, 320. At block 504, the processor 402 may execute a "move bellows" command. Processor-executable instructions stored in the memory 404 may cause the processor 402 to apply power to the motor 222, 322 causing the bellows 220, 320. For example, a dynamic flange 228, 328, may begin movement toward a static flange 229, 329. At block 506, the processor 402 may execute a "check motor current" command. For example, processor-executable instructions stored in the memory 404 may cause the processor 402 to determine an electric current of the motor 222, 322. If the current is steady (i.e., does not rise beyond a threshold rate over time) or does not increase beyond a threshold amount, then the method 500 may return to block 504 and continue bellows movement. If the current rate increase or the current itself exceeds the threshold, then the method 500 may proceed to block 508. At block 508, the processor 402 may execute a "bellows return" command. The distance of this return move may be a constant stored in memory. For example, processor-executable instructions stored in the memory 404 may cause the processor 402 to reverse the motor 222, 322 to a "home" position based on reading pulses from the encoder disk 234, 334. The distance between full compression and "home" also corresponds to the effective counterpulsation volume, as described herein. Finally, at block 510, the pneumatic valves are closed in preparation for counterpulsation pumping to support the patient.

Assuming drive unit 200 and drive unit 300 are configured to move the same amount of air when the corresponding bellows 220, 320 are subjected to linear motion from an expanded to a contracted position, or vice versa (i.e., to deflate or inflate the same size balloon to the effective counterpulsation volume), drive unit 300 may have several advantages over drive unit 200. For example, drive unit 300 can be configured to be smaller and lighter (i.e., have a smaller volume enclosure) than drive unit 200, which can have a significant impact on the ability of a person having to carry drive unit 150 to be mobile/engage in more ambulatory behavior. In particular, bellows 320 can have a smaller bellows travel distance (and a shorter ball screw) than drive unit 220. By increasing the bellows diameter in drive unit 300 relative to the bellows diameter in drive unit 200, (1) the impedance of the pneumatic load on the drive unit (e.g., the collectively pneumatic load of balloon 110, first pneumatic driveline 120, second pneumatic driveline140, and external driveline 172) is better matched to the impedance of the motor (i.e., the mechanical subsystem of drive unit), and (2) the power consumption necessary to inflate and deflate is reduced, thereby increasing efficiency of drive unit 300 as compared to drive unit 200.

With such a configuration the overall dimension of drive unit 300 along its axis of linear motion (i.e., axis B-B) can be smaller than the overall dimension of drive unit 200 along its axis of linear motion (i.e., axis B-B). Whereas the longest dimension of drive unit 200 may be the along its axis of linear motion (i.e., axis B-B), the longest dimension of drive unit 300 may be orthogonal to its axis of linear motion (i.e., orthogonal to axis B-B). This may be particularly true where outer cases 202 and 302 are both a cuboid (or substantially in the shape of a cuboid) and are configured such that the face of static flange 229, 329 is parallel to a face of outer case 202, 302. As depicted in FIG. 3E, the longest dimension of drive unit 300 may correspond to an axis that is parallel to the diameter of bellows 320.

Further, the design of drive unit 300 may be configured to operate fast enough to deflate a corresponding balloon or inflatable member 110 by 50% within approximately 100ms of the R-wave in the QRS complex, so that it may be capable of treating patients with irregular heartbeats (e.g., patients with atrial fibrillation or "a-fib"). As noted above, a-fib is an irregular and/or rapid heart rate that occurs when the two upper chambers of the heart experience chaotic electrical signals. The result is a fast and irregular heart rhythm that is difficult to predict for effective inflation and deflation of the expandable member 110. To effectively treat a-fib patients, the expandable member 110 should be deflated by at least 50% within approximately 100-120ms of the R wave. Without rapid deflation, the expandable member 110 might obstruct blood flow within the aorta and increase the workload of the heart. Using the R-wave in a QRS complex as an indicator of ventricle contraction requires processing of an EKG signal to locate the R-wave, which can consume about 20-50ms, depending on the algorithm employed using conventional algorithms and processors. By setting the ball screw pitch in drive unit 300 between 3.5 and 5.5mm, and using a bellows 320 with an outside diameter between 125 and 111mm with an effective bellow travel distance between 8.0 and 11.5mm, drive unit 300 may be capable of effectively treating patients with irregular heartbeats and a-fib.

Even further, drive unit 300 can be far more efficient than drive unit 200 in terms of power consumption thereby prolonging battery life of the battery (not depicted) powering motor 322 as compared to battery (not depicted) powering motor 222, assuming the same such batteries are the same. In particular, motor 222 may inefficiently use a disproportional amount of power to overcome the rotational inertia of the rotor 222a in drive unit 200 as compared to the amount of power utilized in drive unit 300 to overcome the rotational inertia of rotor 322a. Drive unit 300 realizes this advantage by (a) setting the ball screw pitch in drive unit 300 such that the impedance of the motor 322 is the same or substantially the same (i.e., matched or substantially matched) to the impedance of the pneumatic load on the drive unit 300, and/or (b) using an enlarged bellows 320, which permits a design with a reduced bellows travel (and which may further help match or substantially match impedance of the motor 322 to the pneumatic load on the drive unit 300).

The above-provided tables for drive unit 200 and drive unit 300 are merely exemplary. Drive units with different component values are contemplated as being within the scope of this disclosure.

### Conclusion

The above detailed description of embodiments of the technology are not intended to be exhaustive or to limit the technology to the precise forms disclosed above. Although specific embodiments of, and examples for, the technology are described above for illustrative purposes, various equivalent modifications are possible within the scope of the technology as those skilled in the relevant art will recognize. The various embodiments described herein may also be combined to provide further embodiments.

Unless the context clearly requires otherwise, throughout the description and the examples, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof, means any connection or coupling, either direct or indirect, between two or more elements; the coupling of connection between the elements can be physical, logical, or a combination thereof. Additionally, the words "herein," "above," "below," and words of similar import, when used in this application, shall refer to this application as a whole and not to any particular portions of this application. Where the context permits, words in the above Detailed Description using the singular or plural number may also include the plural or singular number respectively. As used herein, the phrase "and/or" as in "A and/or B" refers to A alone, B alone, and the combination of A and B. It will also be appreciated that specific embodiments have been described herein for purposes of illustration, but that various modifications may be made without deviating from the technology. Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly depicted or described herein.

One, non-limiting embodiment of an invention of the application will now be described by way of reference to the following clauses:
1. A system for limiting inflation of an expandable member in an intravascular circulatory support system of a patient, the system comprising:
   a drive unit including an air mover and a motor, the air mover having a pneumatic output in fluid connection with the expandable member, a first end and a second end, wherein the first end is fixed, and the second end is displaceable; and
   an air mover limiter configured to restrict displacement of the second end to under-inflate the expandable member based on the blood pressure of the patient;
   wherein displacement of the second end moves a volume of air into or out of the expandable member, the volume of air corresponding to the air mover limiter configuration.
2. The system of clause 1, wherein an internal volume of the expandable member is greater than the volume of air.
3. The system of clause 1 or 2, wherein the blood pressure of the patient includes a lowest therapeutic diastolic blood pressure for the patient.
4. The system of clause 3, wherein the first pressure corresponds to an effective counterpulsation volume based on the lowest therapeutic diastolic blood pressure for the patient.
5. The system of any one of the preceding clauses, wherein:
   the motor includes a rotor and a stator;
   a ball nut carrying a ball screw, wherein the ball nut is affixed to the rotor and the ball screw includes a helical raceway.
6. The system of clause 5, wherein:
   the second end is defined by a dynamic flange configured to receive the ball screw; and
   the ball nut includes a plurality of balls that ride within the helical raceway, such that rotation of the rotor causes the plurality of balls to translate such rotation into linear motion of the ball screw within the ball nut, and the second end is fixed to the ball screw.
7. The system of clause 5 or 6 or any one of the preceding clauses, wherein the air mover limiter includes one or more of processor-executable instructions for restricting an output of the air mover and a mechanical restriction for restricting the output of the air mover.
8. The system of clause 5, 6 or 7 or any one of the preceding clauses, wherein the mechanical restriction includes a clutch-like collar shaped to receive the stop pin during rotation of the ball nut in a first direction around the ball screw and to slide under the stop pin during rotation of the ball nut in a second direction around the ball screw.
9. The system of clause 5, 6, 7 or 8 or any one of the preceding clauses, wherein the one or more processor-executable instructions for restricting the output of the air mover includes accessing a home position of an encoder disk coupled to the motor, the home position stored in stored in processor-accessible memory.
10. The system of clause 4 or any one of the preceding clauses, wherein the drive unit includes an encoder disk and an encoder sensor configured to determine one or more of the angular position, speed, and direction of the rotor, and generate one or more positional feedback signals based on the determined one or more of the angular position, speed, and direction of the rotor.
11. The system of clause 10 or any one of the preceding clauses, wherein the air mover limiter includes a processor configured to restrict displacement of the second end further based on the one or more positional feedback signals.
12. The system of clause 11 or any one of the preceding clauses, wherein the processor is further configured to control the motor based on one or more of EKG signals, wherein the one or more EKG signals are of a patient undergoing therapy and one or more pressure signals associated with the pressure within an artery of the patient.
13. The system of clause 1 or any one of the preceding clauses, wherein the processor is further configured to control the motor based on one or more EKG signals, wherein the one or more EKG signals are of a patient undergoing therapy.
14. The system of clause 4 or any one of the preceding clauses, wherein:
   the bellows has an outside diameter,
   the outside diameter of the bellows is selected such that the drive unit can deflate the inflatable member by 50% within approximately 100 to 120 ms of receipt of an R-wave in a QRS complex detected in one or more EKG signals, wherein the one or more EKG signals are of a patient undergoing therapy.
15. The system of clause 14 or any one of the preceding clauses, wherein the outside diameter of the bellows is within the range of approximately 111 mm and 125 mm.
16. The system of clause 1 or any one of the preceding clauses, wherein a travel distance of the bellows is based on the selected outside diameter of the bellows and an effective counterpulsation volume.
17. The system of clause 16 or any one of the preceding clauses, wherein the effective counterpulsation volume is based on a lowest therapeutic diastolic blood pressure for a particular patient, or on an average lowest therapeutic diastolic blood pressure for a patient population.
18. The system unit of clause 5 or any one of the preceding clauses, wherein the pitch of the helical raceway is selected such that the impedance of the motor is the same or substantially the same as the impedance of the pneumatic load on the drive unit.
19. The system unit of clause 1 or any one of the preceding clauses, wherein the diameter of the bellows is selected such that the impedance of the motor is the same or substantially the same as the impedance of the pneumatic load on the drive unit.
20. The system of clause 5 or any one of the preceding clauses, wherein the pitch of the helical raceway is within the range of approximately 3.5 mm and 5.5 mm.

## Claims

1. A system for limiting inflation of an expandable member in an intravascular circulatory support system of a patient, the system comprising:
a drive unit including an air mover and a motor, the air mover having a pneumatic output in fluid connection with the expandable member, a first end and a second end, wherein the first end is fixed, and the second end is displaceable; and
an air mover limiter configured to restrict displacement of the second end to under-inflate the expandable member based on the blood pressure of the patient;
wherein displacement of the second end moves a volume of air into or out of the expandable member, the volume of air corresponding to the air mover limiter configuration.

2. The system of claim 1, wherein an internal volume of the expandable member is greater than the volume of air.

3. The system of claim 1, wherein the blood pressure of the patient includes a lowest therapeutic diastolic blood pressure for the patient.

4. The system of claim 3, wherein the first pressure corresponds to an effective counterpulsation volume based on the lowest therapeutic diastolic blood pressure for the patient.

5. The system of claim 1, wherein:
the motor includes a rotor and a stator;
a ball nut carrying a ball screw, wherein the ball nut is affixed to the rotor and the ball screw includes a helical raceway.

6. The system of claim 5, wherein:
the second end is defined by a dynamic flange configured to receive the ball screw; and
the ball nut includes a plurality of balls that ride within the helical raceway, such that rotation of the rotor causes the plurality of balls to translate such rotation into linear motion of the ball screw within the ball nut, and the second end is fixed to the ball screw.

7. The system of claim 6, wherein the air mover limiter includes one or more of processor-executable instructions for restricting an output of the air mover and a mechanical restriction for restricting the output of the air mover.

8. The system of claim 7, wherein the mechanical restriction includes a clutch-like collar shaped to receive the stop pin during rotation of the ball nut in a first direction around the ball screw and to slide under the stop pin during rotation of the ball nut in a second direction around the ball screw.

9. The system of claim 7, wherein the one or more processor-executable instructions for restricting the output of the air mover includes accessing a home position of an encoder disk coupled to the motor, the home position stored in stored in processor-accessible memory.

10. The system of claim 4, wherein the drive unit includes an encoder disk and an encoder sensor configured to determine one or more of the angular position, speed, and direction of the rotor, and generate one or more positional feedback signals based on the determined one or more of the angular position, speed, and direction of the rotor.

11. The system of claim 10, wherein a) the air mover limiter includes a processor configured to restrict displacement of the second end further based on the one or more positional feedback signals; or b) the air mover limiter includes a processor configured to restrict displacement of the second end further based on the one or more positional feedback signals and wherein the processor is further configured to control the motor based on one or more of EKG signals, wherein the one or more EKG signals are of a patient undergoing therapy and one or more pressure signals associated with the pressure within an artery of the patient.

12. The system of claim 1, wherein the processor is further configured to control the motor based on one or more EKG signals, wherein the one or more EKG signals are of a patient undergoing therapy.

13. The system of claim 4, wherein:
a) the bellows has an outside diameter,
the outside diameter of the bellows is selected such that the drive unit can deflate the inflatable member by 50% within approximately 100 to 120 ms of receipt of an R-wave in a QRS complex detected in one or more EKG signals, wherein the one or more EKG signals are of a patient undergoing therapy; or
b) the bellows has an outside diameter,
the outside diameter of the bellows is selected such that the drive unit can deflate the inflatable member by 50% within approximately 100 to 120 ms of receipt of an R-wave in a QRS complex detected in one or more EKG signals, wherein the one or more EKG signals are of a patient undergoing therapy and wherein the outside diameter of the bellows is within the range of approximately 111 mm and 125 mm.

14. The system of claim 1, wherein a) a travel distance of the bellows is based on the selected outside diameter of the bellows and an effective counterpulsation volume; or b) a travel distance of the bellows is based on the selected outside diameter of the bellows and an effective counterpulsation volume and wherein the effective counterpulsation volume is based on a lowest therapeutic diastolic blood pressure for a particular patient, or on an average lowest therapeutic diastolic blood pressure for a patient population; or c) the diameter of the bellows is selected such that the impedance of the motor is the same or substantially the same as the impedance of the pneumatic load on the drive unit.

15. The system unit of claim 5, wherein the pitch of the helical raceway is selected such that the impedance of the motor is the same or substantially the same as the impedance of the pneumatic load on the drive unit, or wherein the pitch of the helical raceway is within the range of approximately 3.5 mm and 5.5 mm.
